# EUROPEAN PATENT APPLICATION

(11) **EP 4 101 455 A1**
(43) Date of publication of application: **14.12.2022**
(21) Application number: 21738224.1
(22) Date of filing: 07.01.2021
(51) Int. Cl.: A61K 33/42, A61K 9/14, A61P 1/04, A61P 7/04, A61P 17/02

(54) **PLATELET AGGREGATOR**

(30) Priority: 08.01.2020 JP 2020001319
(71) Applicant: Kamui Pharma, Inc., Hokkaido 078-8802 (JP); National University Corporation Asahikawa Medical University, Asahikawa, Hokkaido 078-8510 (JP)
(72) Inventor: FUJIYA Mikihiro, Asahikawa, Hokkaido 078-8510 (JP); KONISHI Hiroaki, Asahikawa, Hokkaido 078-8510 (JP); TANAKA Hiroki, Asahikawa, Hokkaido 078-8510 (JP); ISOZAKI Shotaro, Asahikawa, Hokkaido 078-8510 (JP); OMACHI Yoshihiro, Osaka-shi, Osaka 532-0024 (JP); OGAWA Naoki, Asahikawa, Hokkaido 078-8510 (JP)
(74) Representative: Carpmaels & Ransford LLP
(86) International application number: PCT/JP2021/000261
(87) International publication number: WO 2021/141066

(57) **Abstract**

The present invention relates to a platelet aggregating agent containing amorphous polyphosphate as an active ingredient, wherein the polyphosphate is a Ca salt of polyphosphate. The platelet aggregating agent acts on damaged gastrointestinal mucosa in inflammatory bowel disease and exerts a platelet aggregating action, thereby allowing remission/improvement of the inflammatory bowel disease.

## Description

### Technical Field

### [Related Applications]

The present application claims priority based on Japanese Patent Application No. 2020-001319 (filed on January 8, 2020), the contents of which are incorporated herein by reference.

### [Technical Field]

The present invention relates to a platelet aggregating agent having a Ca salt of polyphosphate as an active ingredient. More specifically, it relates to a medicament which acts on damaged gastrointestinal mucosa in inflammatory bowel disease and exerts a platelet aggregating action, thereby allowing remission/improvement of the inflammatory bowel disease.

### Background Art

Although anti-inflammatory agents are used as the standard therapeutic agent for inflammatory bowel disease (IBD), as typified by ulcerative colitis (UC) and Crohn's disease (CD), there are no drugs which directly induce mucosal healing. Whereas conventional anti-inflammatory agents have been used to treat IBD symptomatically, recently, the true goal of the treatment of IBD is considered to be "mucosal healing". Probiotics, as typified by lactic acid bacteria, are highly safe by their long history of use as a food and are known to have a certain action of regulating the intestines and the like. However, the mechanism of action of probiotics on intestinal condition is still unclear in many respects. To elucidate this, the bioactive molecules produced by probiotics are being identified and analyzed.

The inventors have identified a long-chain polyphosphate derived from Lactobacillus brevis as a molecule that strengthens the intestinal barrier function. They then demonstrated that long-chain polyphosphate improves impaired intestinal tract barrier function and intestinal tract injury caused by DSS treatment (Patent Literature 1). In addition, they demonstrated that long-chain polyphosphate produces mucosal healing in patients with refractory ulcerative colitis (Non Patent Literature 1).

Polyphosphate is known to have wound-healing and anti-inflammatory properties, and the development of wound dressings and dental materials using an amorphous substance or nanoparticles composed of calcium polyphosphate salt is anticipated (Patent Literature 2).

Polyphosphate is also known as an activator of blood coagulation. When released from platelets, polyphosphate activates factor XII protease in the blood, which elicits a clotting reaction. Various salts of natural polyphosphate, such as Ca, Mg, Na, and K salts, have been postulated, and amorphous nanoparticles are also known to exist (Non Patent Literatures 2 and 3). Donovan et al. prepared nanoparticles from polyphosphates of different chain lengths and reported its influence on the size of polyphosphate and blood coagulation (Non Patent Literature 4). However, there are no reports on amorphous or nanoparticles of polyphosphate, particularly on the influence of differences in their salts on platelet aggregation and inflammatory bowel disease.

### Citation List

### Patent Literature

Patent Literature 1: International Publication No. WO 2011/125619
Patent Literature 2: International Publication No. WO 2016/079006

### Non Patent Literature

Non Patent Literature 1: Fujita et al., "Long-Chain Polyphosphate Is a Potential Agent for Inducing Mucosal Healing of the Colon in Ulcerative Colitis." Clin Pharmacol Ther. 2019 Sep.
Non Patent Literature 2: Feng et al., "Biogenic Polyphosphate Nanoparticles from a Marine Cyanobacterium Synechococcus sp. PCC 7002: Production, Characterization, and Anti-Inflammatory Properties In Vitro." Mar Drugs. 2018 Sep 10;16(9).
Non Patent Literature 3: Feng et al., "Biogenic Polyphosphate Nanoparticles from Synechococcus sp. PCC 7002 Exhibit Intestinal Protective Potential in Human Intestinal Epithelial Cells In Vitro and Murine Small Intestine Ex Vivo." J Agric Food Chem. 2018 Aug 1;66(30):8026-8035.
Non Patent Literature 4: Donovan et al., "Size-controlled synthesis of granular polyphosphate nanoparticles at physiologic salt concentrations for blood clotting." Biomacromolecules. 2014 Nov 10;15(11):3976-84

### Summary of Invention

### Technical Problem

The present invention addresses the problem of elucidating the bioactivity of polyphosphate and optimizing the molecular design to maximize its activity.

### Solution to Problem

The inventors have found that the platelet aggregating action of polyphosphate (salt) is selective for calcium (Ca) salts and was not observed for other metal salts such as sodium (Na) salts, and that this effect of polyphosphate Ca salts is exerted on damaged gastrointestinal mucosa in inflammatory bowel disease to promote healing of the mucosa.

The present invention is based on the above findings, and relates to the following (1) to (9):
(1) A platelet aggregating agent comprising amorphous polyphosphate as an active ingredient, wherein the polyphosphate is a Ca salt of polyphosphate.
(2) The platelet aggregating agent according to (1), which promotes platelet aggregation in damaged gastrointestinal mucosa.
(3) The platelet aggregating agent according to (1) or (2), which is administered to a patient with inflammatory bowel disease.
(4) The platelet aggregating agent according to any one of (1) to (3), which is orally administered.
(5) The platelet aggregating agent according to any one of (1) to (4), which promotes healing of damaged gastrointestinal mucosa.
(6) The platelet aggregating agent according to any one of (1) to (5), wherein the amorphous polyphosphate is in nanoparticle form.
(7) The platelet aggregating agent according to (6), wherein a zeta potential of the polyphosphate nanoparticles is 0 mV or less. The zeta potential of polyphosphate is preferably -20 mV or less, and more preferably -30 mV or less.
(8) The platelet aggregating agent according to (6) or (7), wherein an average particle size of the polyphosphate nanoparticles is 10 nm to 3000 nm.
(9) The platelet aggregating agent according to any one of (1) to (8), wherein the average chain length of the polyphosphate is 5 phosphate units or more, preferably 100 phosphate units or more, and even more preferably 300 phosphate units or more.

### Advantageous Effects of Invention

The polyphosphate of the present invention is composed of an amorphous substance or nanoparticles composed of a polyphosphate Ca salt, which is the main body of the platelet aggregating action. Therefore, a higher platelet aggregating action can be expected compared to natural polyphosphate. By oral administration, the polyphosphate of the present invention acts on damaged gastrointestinal mucosa and exerts an excellent platelet aggregating action, thereby allowing improvement/remission of inflammatory bowel disease.

### Brief Description of Drawings

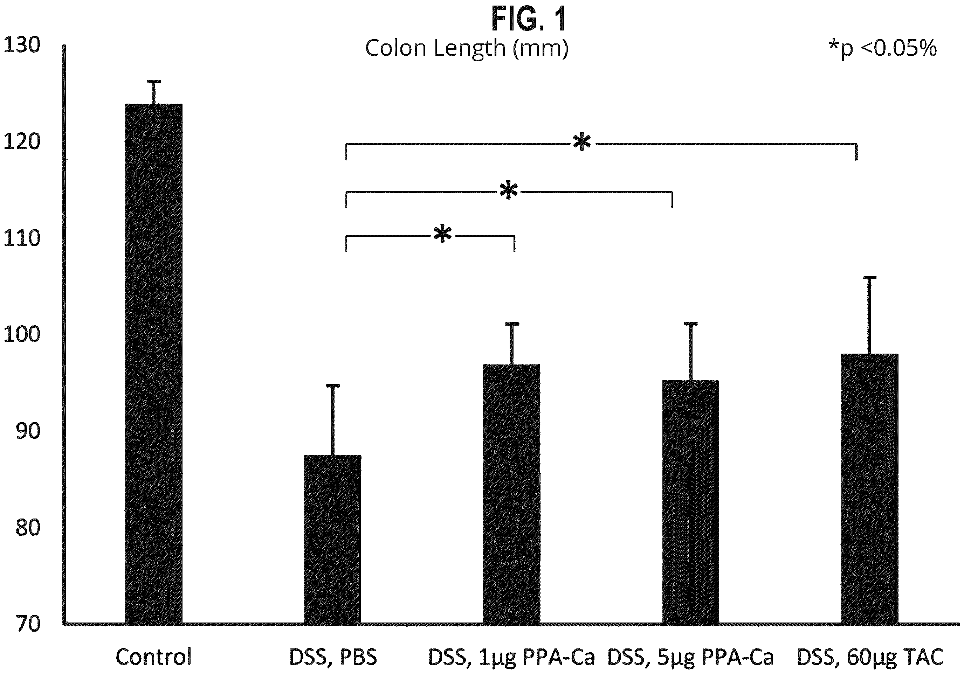

[Figure 1] Figure 1 shows the lengths of mice intestinal tract (n=8 each). Normal mice (Control), PBS-treated acute enterocolitis mice group (DSS, PBS), calcium polyphosphate-treated (1 µg/mouse) acute enterocolitis mice group(DSS, 1 µg PPA-Ca), calcium polyphosphate-treated (5 µg/mouse) acute enterocolitis mice group (DSS, 5 µg PPA-Ca), and tacrolimus-treated (60 µg/mouse) group (DSS, 60 µg TAC).

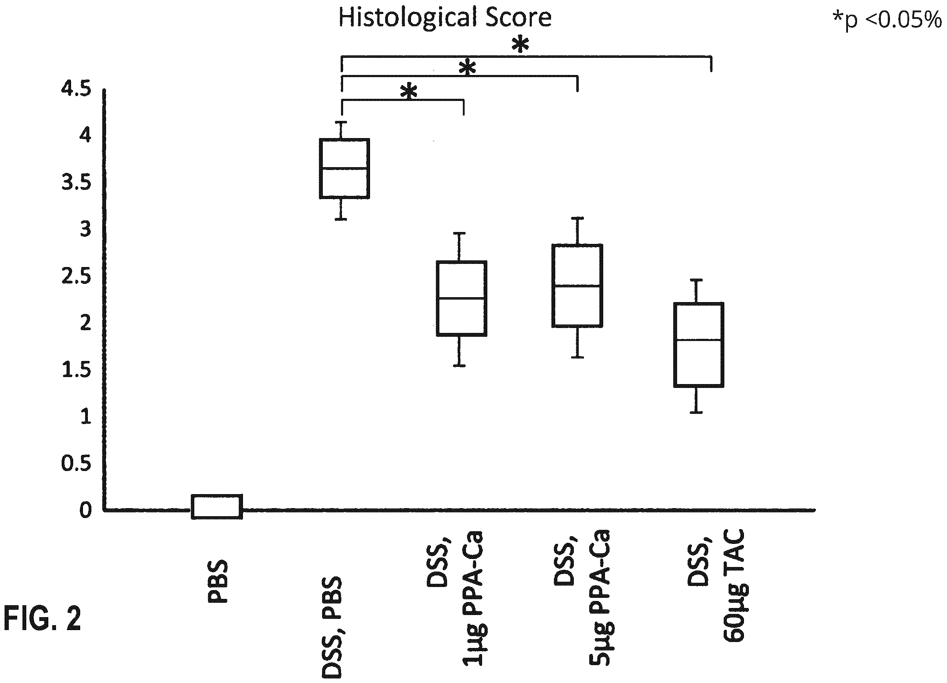

[Figure 2] Figures 2 shows the histological severities (n=8 each). Normal mice (PBS), PBS-treated acute enterocolitis mice group (DSS, PBS), calcium polyphosphate-treated (1 µg/mouse) acute enterocolitis mice group(DSS, 1 µg PPA-Ca), calcium polyphosphate-treated (5 µg/mouse) acute enterocolitis mice group (DSS, 5 µg PPA-Ca), and tacrolimus-treated (60 µg/mouse) group (DSS, 60 µg TAC).

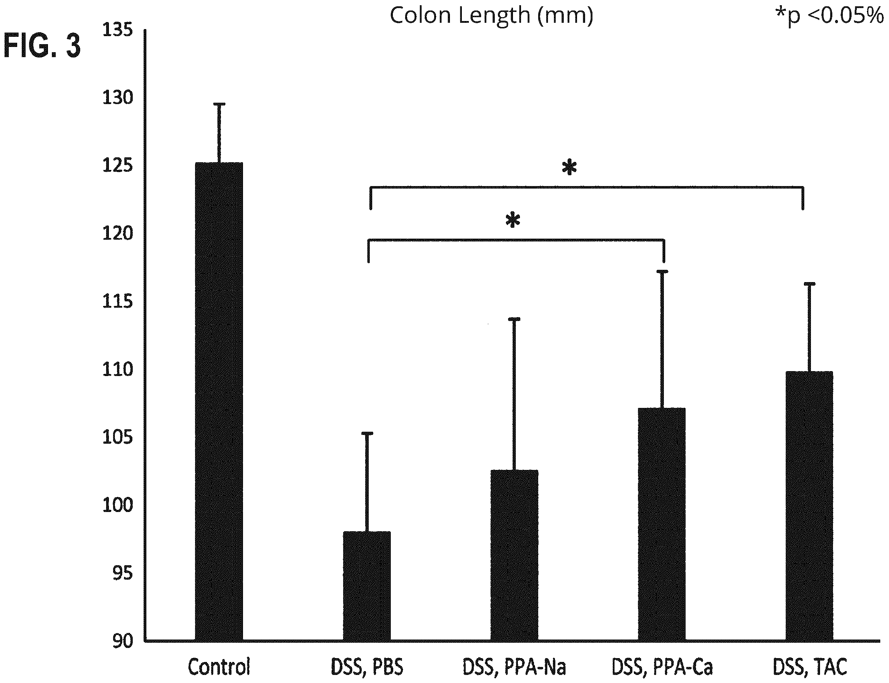

[Figure 3] Figure 3 shows the lengths of mice intestinal tract (normal mice; n=6, PBS-treated acute enterocolitis mice group; n=9, sodium polyphosphate-treated acute enterocolitis mice group; n=7, calcium polyphosphate-treated acute enterocolitis mice group; n=8, and tacrolimus-treated group; n=5). Normal mice (Control), PBS-treated acute enterocolitis mice group (DSS, PBS), sodium polyphosphate-treated (5 µg/mouse) acute enterocolitis mice group (DSS, PPA-Na), calcium polyphosphate-treated (5 µg/mouse) acute enterocolitis mice group (DSS, PPA-Ca), and tacrolimus-treated (60 µg/mouse) group (DSS, TAC).

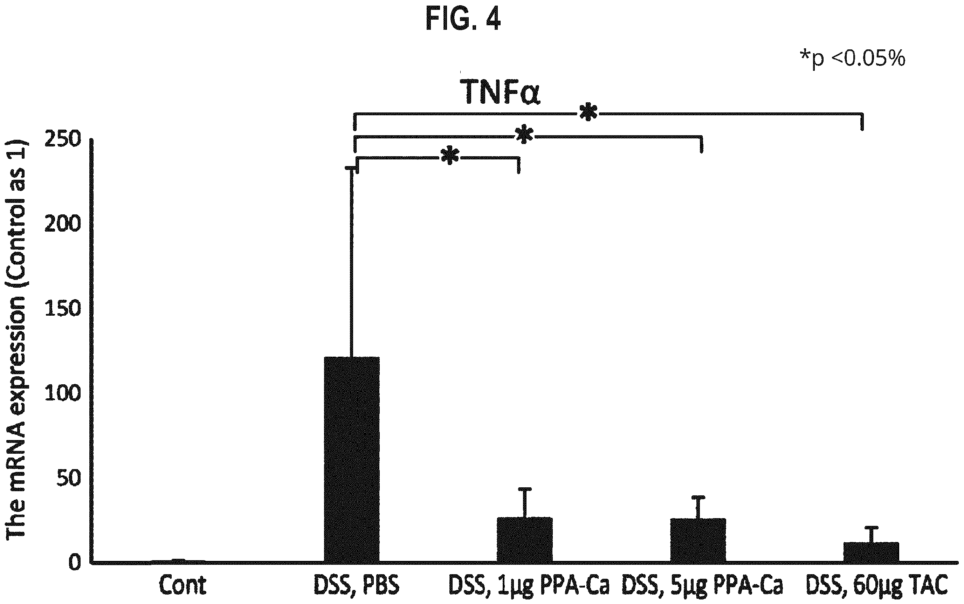

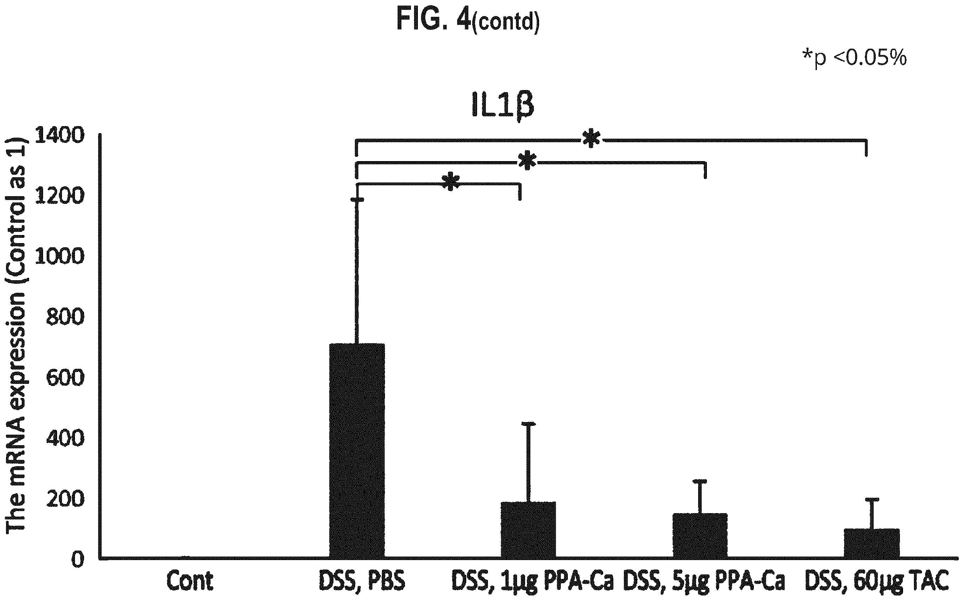

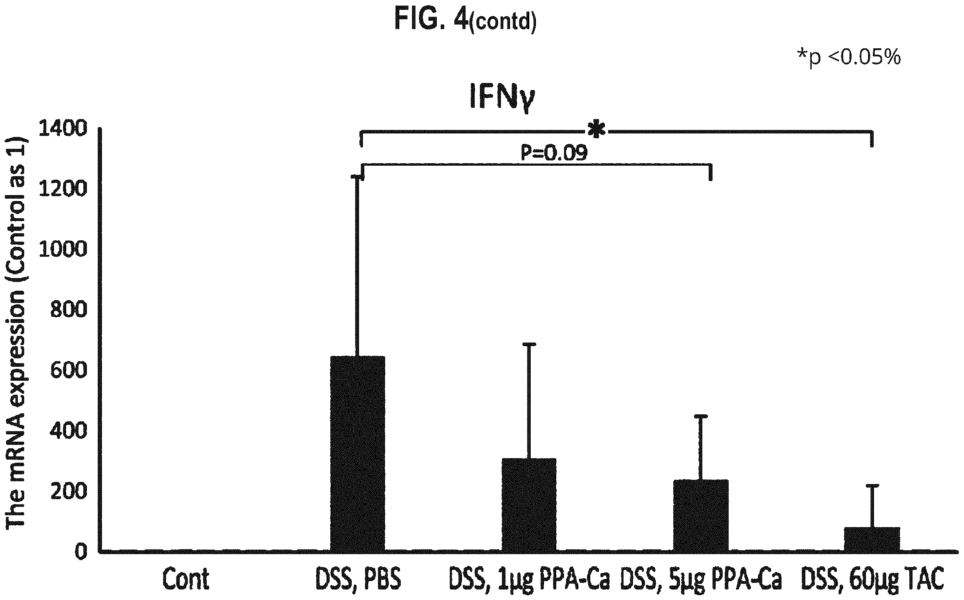

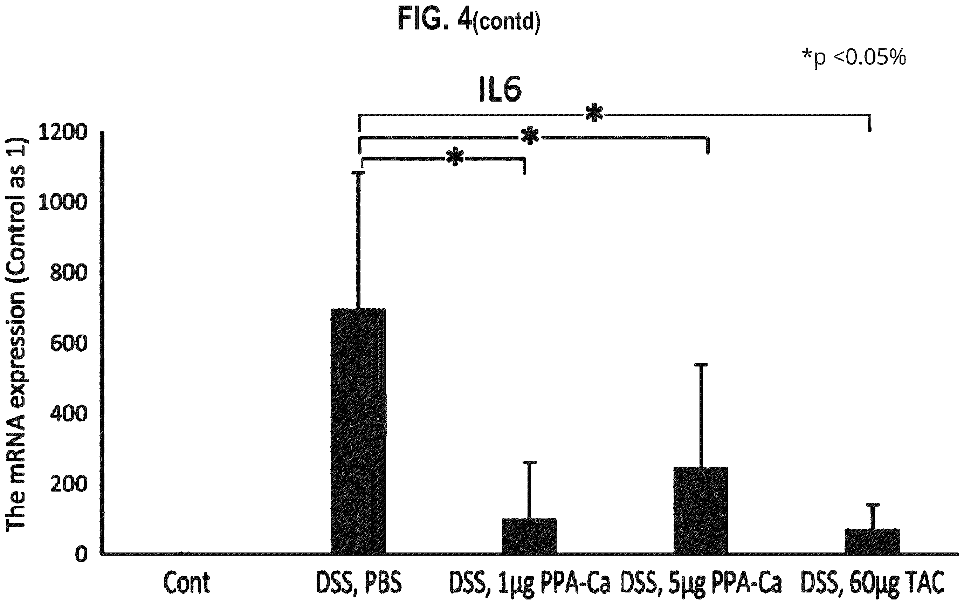

[Figure 4] Figure 4 shows the results of expression analysis of inflammatory cytokines (TNFα, IL-1β, IFNγ, and IL6) by RT-PCR (n=6 to 8). The graph is, from the left, normal mice (Control), PBS-treated acute enterocolitis mice group (DSS, PBS), calcium polyphosphate-treated (1 µg/mouse) acute enterocolitis mice group (DSS, 1 µg PPA-Ca), calcium polyphosphate-treated (5 µg/mouse) acute enterocolitis mice group (DSS, 5 µg PPA-Ca), and tacrolimus-treated (60 µg/mouse) group (DSS, 60 µg TAC).

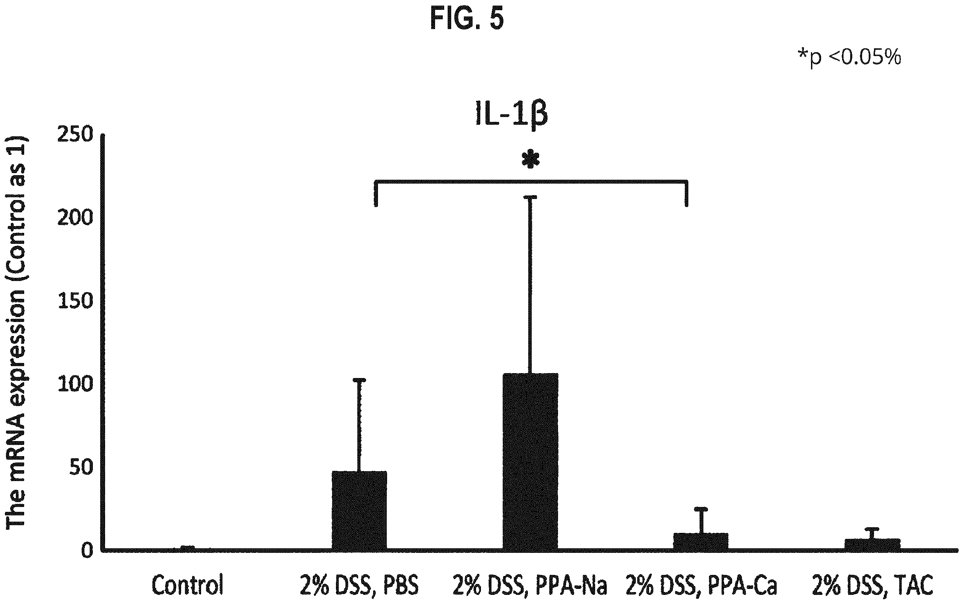

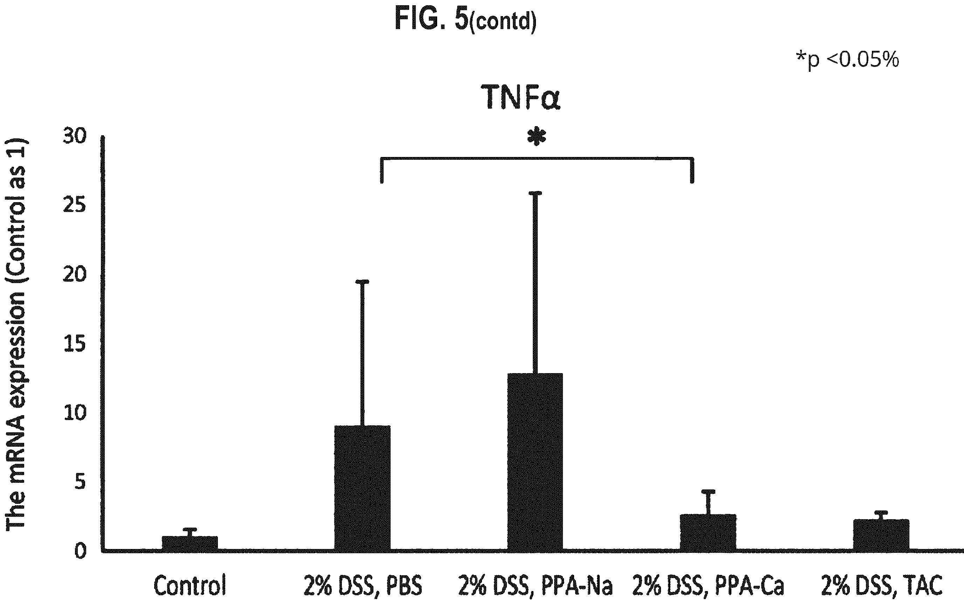

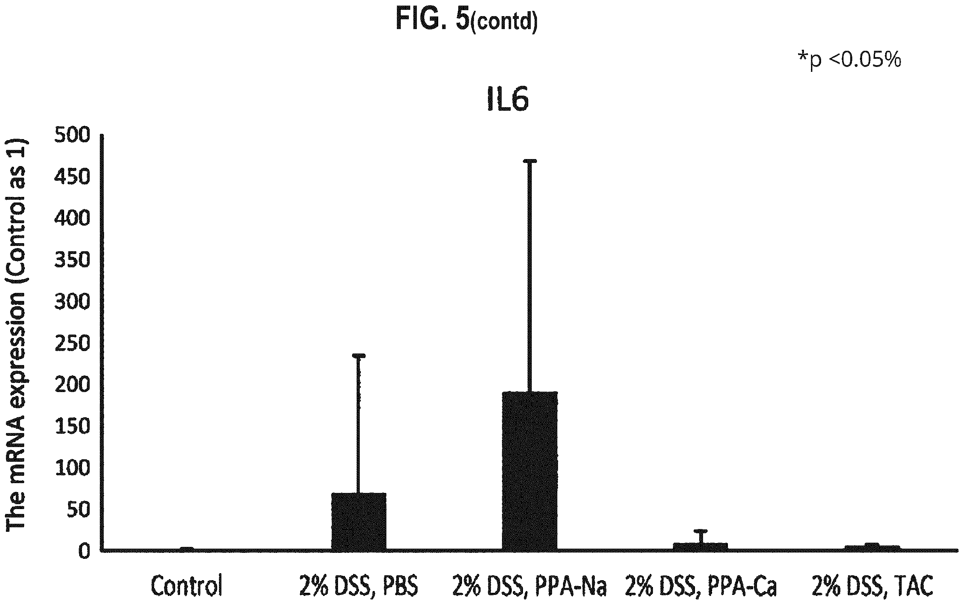

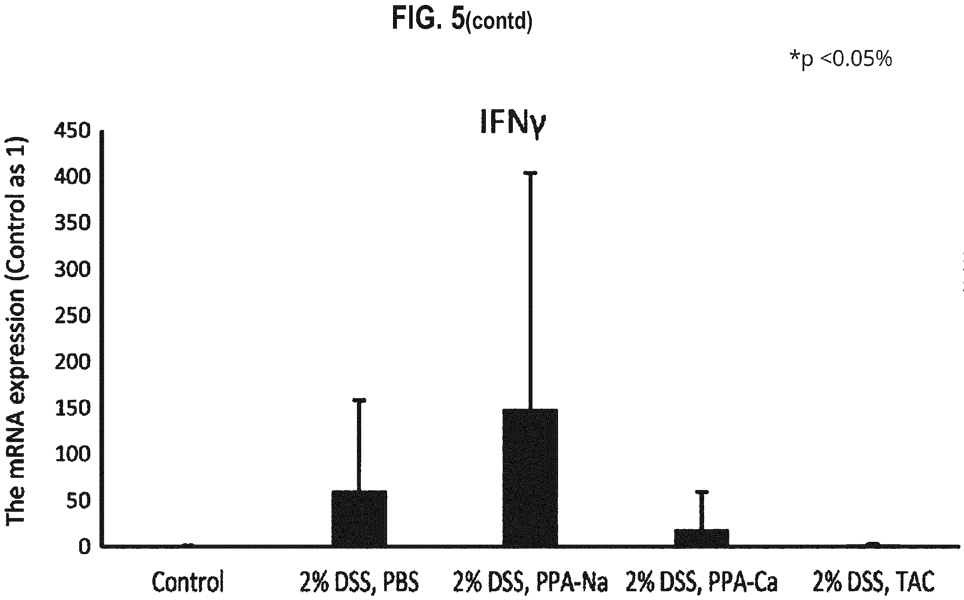

[Figure 5] Figure 5 shows the results of expression analysis of inflammatory cytokines (TNFα, IL-1β, IFNγ, and IL6) by RT-PCR (n=5 to 9). Normal mice (Control), PBS-treated acute enterocolitis mice group (DSS, PBS), sodium polyphosphate-treated (5 µg/mouse) acute enterocolitis mice (DSS, PPA-Na), calcium polyphosphate-treated (5 µg/mouse) acute enterocolitis mice group (DSS, PPA-Ca), tacrolimus-treated (60 µg/mouse) group (DSS, TAC).

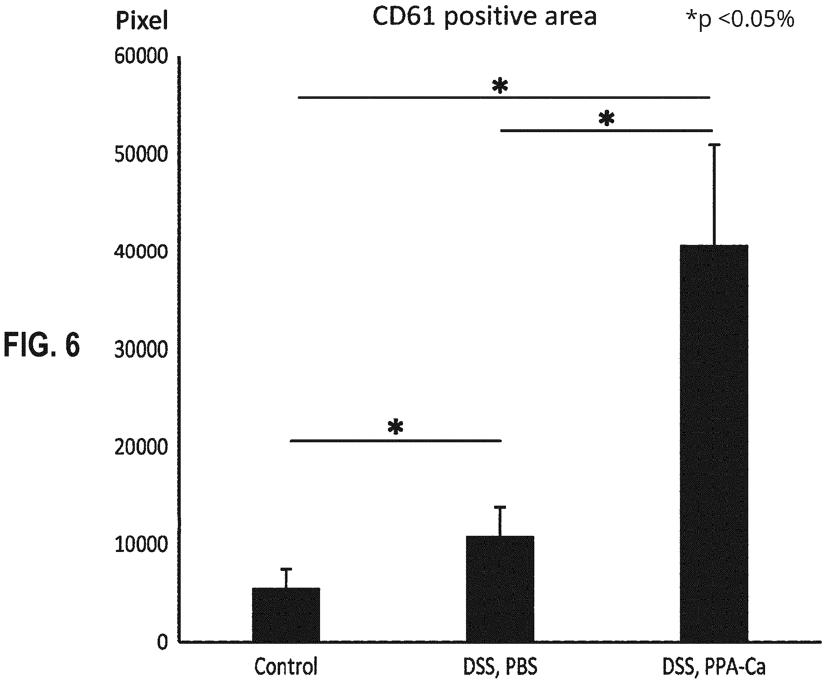

[Figure 6] Figure 6 shows the CD61-positive areas (pixel) (n=7). The graph is, from the left, normal mice (Control), PBS-treated acute enterocolitis mice group (DSS, PBS), and calcium polyphosphate-treated (5 µg/mouse) acute enterocolitis mice group (DSS, PPA-Ca).

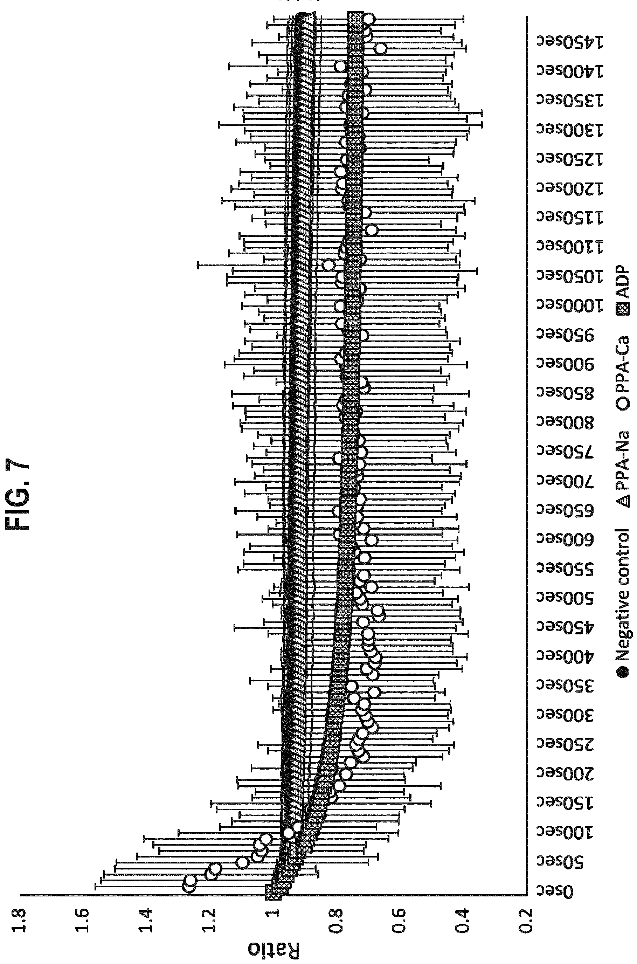

[Figure 7] Figure 7 shows the time course of the turbidity of the solution (n=5). Sodium polyphosphate (PPA-Na) shows little difference from the results of no addition (Negative control). On the other hand, calcium polyphosphate (PPA-Ca) showed a decrease in the turbidity similar to that of adenosine diphosphate (ADP), although the turbidity temporarily increased immediately after addition.

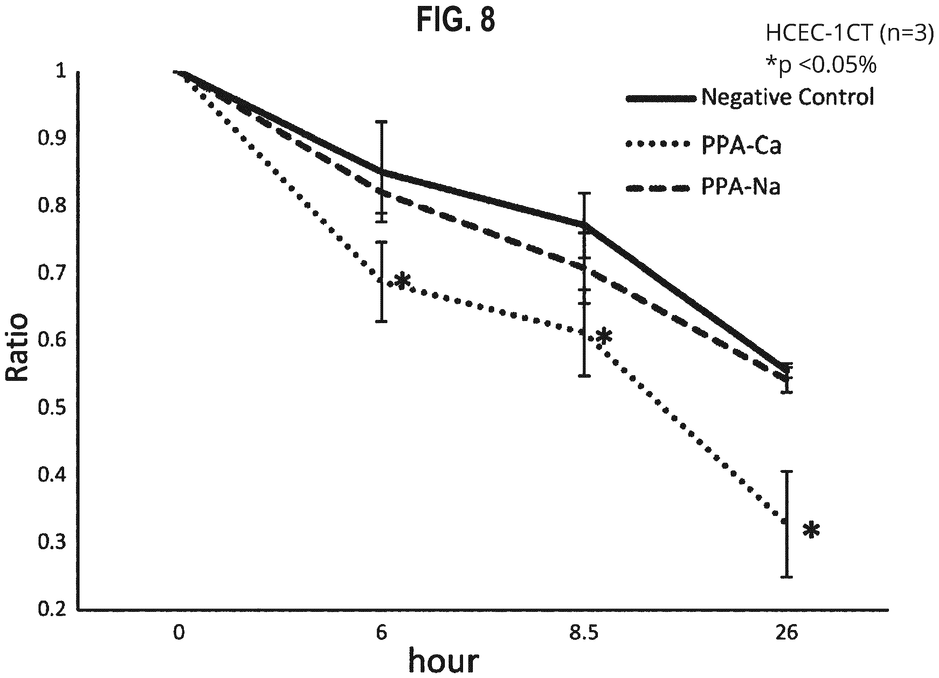

[Figure 8] Figure 8 shows the time course of wound width (n=3). The graph is, from the top, no addition (Negative control), sodium polyphosphate (PPA-Na), and calcium polyphosphate (PPA-Ca).

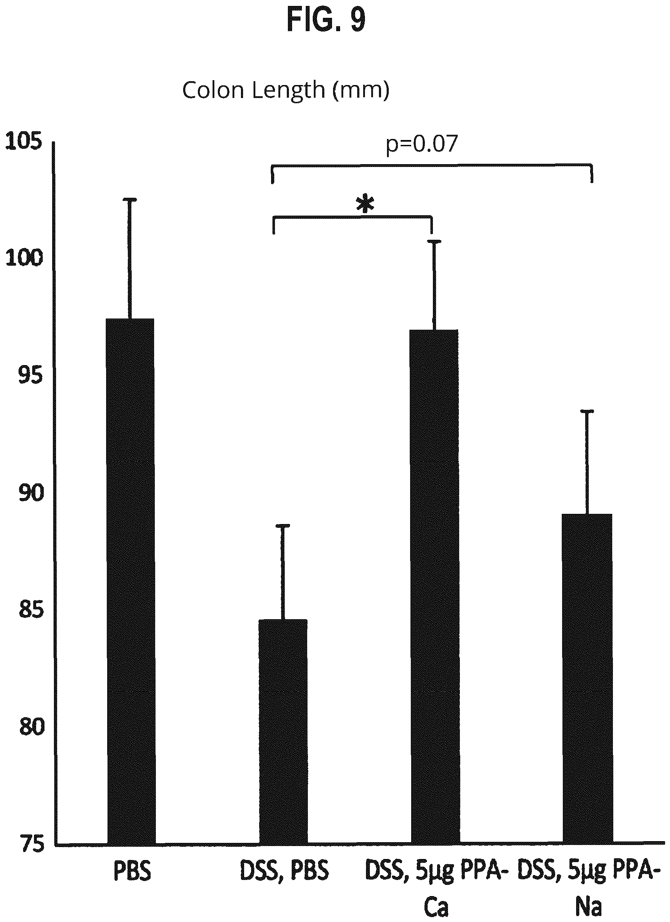

[Figure 9] Figure 9 shows the lengths of mice intestinal tract (normal mice; n=5, PBS-treated chronic enterocolitis mice group, calcium polyphosphate-treated chronic enterocolitis mice group, and sodium polyphosphate-treated chronic enterocolitis mice group; n=7). Normal mice (Control), PBS-treated chronic enterocolitis mice group (DSS, PBS), calcium polyphosphate-treated (5 µg/mouse) chronic enterocolitis mice group (DSS, 5 µg PPA-Ca), and sodium polyphosphate-treated (5 µg/mouse) chronic enterocolitis mice group (DSS, 5 µg PPA-Na).

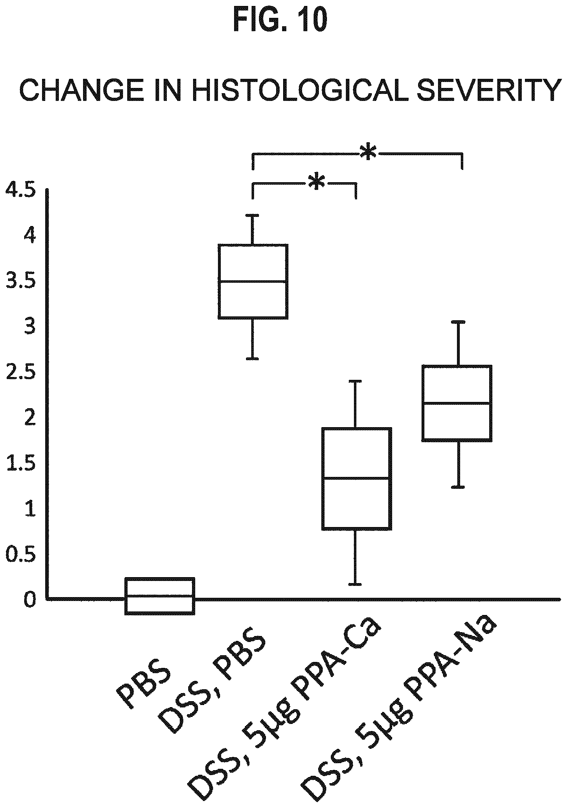

[Figure 10] Figure 10 shows the histological severity (normal mice; n=5, PBS-treated chronic enterocolitis mice group, calcium polyphosphate-treated chronic enterocolitis mice group, and sodium polyphosphate-treated chronic enterocolitis mice group; n=7). Normal mice (Control), PBS-treated chronic enterocolitis mice group (DSS, PBS), calcium polyphosphate-treated (5 µg/mouse) chronic enterocolitis mice group (DSS, 5 µg PPA-Ca), and sodium polyphosphate-treated (5 µg/mouse) chronic enterocolitis mice group (DSS, 5 µg PPA-Na).

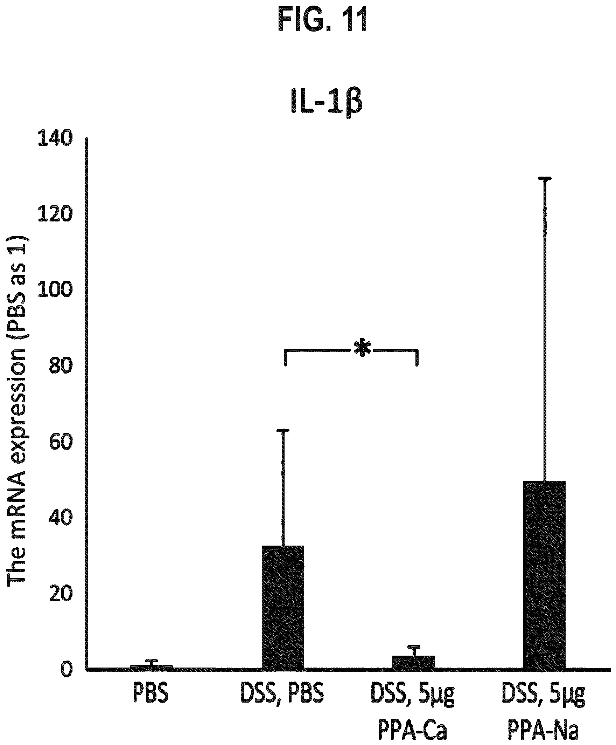

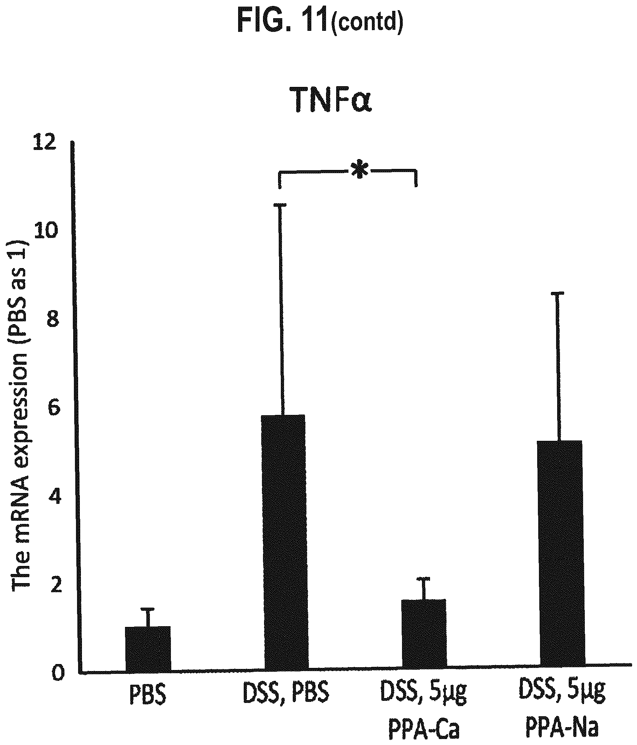

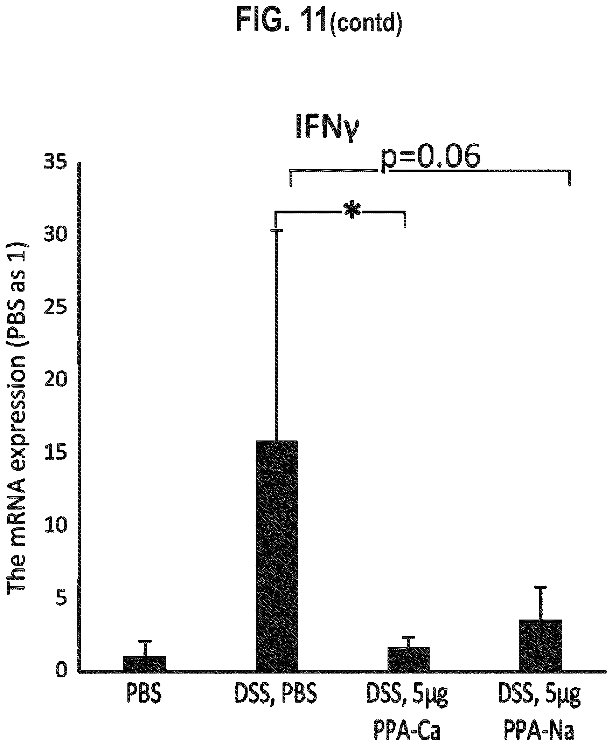

[Figure 11] Figure 11 shows the results of expression analysis of inflammatory cytokines (IL-1β, TNFα, and IFNy) by RT-PCR (normal mice; n=5, PBS-treated chronic enterocolitis mice group, calcium polyphosphate-treated chronic enterocolitis mice group, and sodium polyphosphate-treated chronic enterocolitis mice group; n=7). The graph is, from the left, normal mice (Control), PBS-treated chronic enterocolitis mice group (DSS, PBS), calcium polyphosphate-treated (5 µg/mouse) chronic enterocolitis mice group(DSS, 5 µg PPA-Ca), and sodium polyphosphate-treated (5 µg/mouse) chronic enterocolitis mice group (DSS, 5 µg PPA-Na).

### Description of Embodiments

### 1. Polyphosphate

In the present description, "polyphosphate" means a condensed phosphate compound in which phosphoric acid (H₃PO₄) is condensed by dehydration, and may be a chain, cyclic, or have branching.

In the present description, the number (n) of "phosphate units" contained in the linear portion of polyphosphate is used to indicate the average chain length of polyphosphate. For example, an average chain length of 10 phosphate units (n=10) or more means that the number of repeating phosphate units contained in the linear portion of the polyphosphate is 10 or more.

The average chain length of the polyphosphate of the present invention is preferably 5 phosphate units or more, more preferably 100 phosphate units or more, and even more preferably 300 phosphate units or more. Using a longer-chain polyphosphate allows to improve not only the platelet aggregating action but also the barrier function strengthening action, and to more effectively prevent or improve (heal, alleviate) inflammatory bowel disease.

The polyphosphate is preferably a high molecular weight polyphosphate which does not permeate an ultrafiltration membrane with a molecular weight cut off of 10 kDa when ultrafiltration is performed. Ultrafiltration means to filter out molecules in a sample to be filtered by using a spin column equipped with an ultrafiltration membrane consisting of PES (polyethersulfone), adding the sample to be filtered to this spin column, and then subjecting centrifugation.

Polyphosphate may be chemically synthesized, synthesized in vitro using biomolecules such as enzymes, or synthesized using microorganisms and the like which produce polyphosphate. When synthesizing linear polyphosphate, it is preferable to synthesize it in vitro using biomolecules such as enzymes, or to synthesize it by using microorganisms and the like, as linear polyphosphate can be obtained with high efficiency.

Examples of the method for chemically synthesizing polyphosphate include a method in which a reaction solution containing ATP as a raw material is heated, then condensed by dehydration. The heating temperature can be, for example, 150 to 350°C.

Examples of the method for synthesizing polyphosphate in vitro using biomolecules such as enzymes include a method in which polyphosphate kinase (PPK), an enzyme that synthesizes polyphosphate, is used as the "biomolecule such as an enzyme", and polyphosphate is synthesized by the enzymatic action of PPK using ATP as the raw material. PPK has been reported to be carried by many probiotics such as the Lactobacillus rhamnosus GG strain and strains belonging to Lactobacillus brevis, and the gene sequence of the enzyme has been published in DB.

PPK can be obtained from any strain expressing PPK or purchased commercially, as long as it is capable of synthesizing polyphosphate using ATP as a substrate. The PPK can be, for example, a PPK derived from Propionibacterium shermanii.

The enzymatic reaction by PPK is reversible, but if a large amount of ADP is present in the reaction solution compared to ATP, the degradation reaction of polyphosphate will become dominant so that the ADP/ATP ratio reaches equilibrium. Therefore, to efficiently synthesize polyphosphate, it is preferable not to add ADP to the reaction solution. The other conditions such as the composition of the reaction solution, the reaction temperature, and the reaction time can be set as appropriate to optimize it for the PPK activity and according to the synthesis scale and the like. As an example, the reaction conditions for the case of synthesizing polyphosphate using PPK derived from Propionibacterium shermanii are shown below. First, the composition of the reaction solution can be 50 mM Tris-HCI (pH 7.4), 40 mM ammonium sulfate, 4 mM MgCl₂, 40 mM phosphocreatine, 20 ng/ml creatine kinase, 1 mM ATP (pH 7.2) and 1 U/ml PKK, the reaction temperature can be 37°C, and the reaction time can be 0.5 to 10 hours. The reaction time is set as appropriate according to the molecular weight and yield of the target polyphosphate. For example, to obtain a high molecular weight polyphosphate in good yield, the reaction time is preferably 1 to 5 hours.

Examples of the method for synthesizing by using microorganisms include a method in which microorganisms producing polyphosphate are cultured under the appropriate culture conditions and made to produce polyphosphate. Examples of microorganisms producing polyphosphate include the Lactobacillus rhamnosus GG strain, Lactobacillus brevis SBC8803 strain, strains belonging to Lactobacillus, strains belonging to Bifidobacterium, strains belonging to Enterococcus, strains belonging to Lactococcus, strains belonging to Pediococcus, strains belonging to Leuconostoc, strains belonging to Streptococcus, strains belonging to Bacteroides, strains belonging to Eubacterium, and strains belonging to Clostridium.

Polyphosphate can be synthesized by culturing microorganisms in an appropriate culture medium capable of sustaining the growth of the microorganisms used and under appropriate culture temperature conditions. The synthesized polyphosphate can be recovered from the culture medium after culture or by crushing the microorganisms after culture.

In the purification step of the synthesized polyphosphate, purification methods widely used in the present art, such as size exclusion chromatography, ion exchange chromatography, affinity chromatography, high-performance liquid chromatography (HPLC), dialysis, salting out, ammonium sulfate precipitation, precipitation, and crystallization, can be used in combination as appropriate. The purification method to be used can be determined as appropriate according to the method used in the polyphosphate synthesis step, the target degree of purification, the target yield, and the like.

In the present invention, the polyphosphate is an amorphous substance composed of a Ca salt of polyphosphate. A high effect can be expected as amorphous polyphosphate Ca salt is insoluble in water, and thus reaches the intestinal mucosa, the site of action, as a solid, and is taken up by epithelial cells through endocytosis. An amorphous polyphosphate Ca salt insoluble in water simplifies the API manufacturing process, including such as recovery, washing, and drying, and therefore is also advantageous in terms of manufacturing cost.

In the present invention, the amorphous polyphosphate Ca salt is preferably in nanoparticle form. In the present description, a "nanoparticle" means a particle whose size is on the order of nanometers. The polyphosphate nanoparticles of the present invention are particles having an average particle size of 10 nm to 3000 nm and have a value of a charged zeta potential of 0 mV or less, preferably -20 mV or less, and more preferably -30 mV or less. The average particle size and zeta potential mean the average particle size and zeta potential obtained by dynamic light scattering/photon correlation (particle size distribution measurement) and laser Doppler multipoint detection electrophoresis (zeta potential measurement). The nanoparticles have a smooth sphere-like shape with relatively uniform particle size, and therefore have physical properties preferable in terms of formulation.

The nanoparticles composed of a Ca salt of polyphosphate can be produced, for example, as follows. A sodium polyphosphate salt is dissolved in water or a buffer solution (for example, Tris-HCI buffer or carbonate-bicarbonate buffer) and pH is adjusted (for example, pH 10). To this, CaCl₂ is added and stirred while maintaining the pH with NaOH to flocculate as a Ca salt of polyphosphate, and the precipitate is dried then passed through a sieve of a proper size.

As shown in the Examples below, the inventors have found that a Ca salt of polyphosphate promotes platelet aggregation in the damaged intestinal mucosa of inflammatory bowel disease, and allows to promote the improvement, remission, and healing of the damaged mucosa. This effect of promoting platelet aggregation is not observed with other metal salts of polyphosphate, such as polyphosphate Na, and can be considered as an effect unique to Ca salts.

### 2. Platelet aggregating agent

The "platelet aggregating agent" according to the present invention means a substance having an action of aggregating platelets. The platelet aggregating agent of the present invention is characterized in that it contains the amorphous Ca salt of polyphosphate described above as an active ingredient and is capable of aggregating platelets in gastrointestinal mucosa incurred damage.

In living organisms, polyphosphate is also known as an activator of blood coagulation. Polyphosphate is stored in platelet dense granules and, when released in response to stimulation, activates factor XII protease in the blood, which elicits a clotting reaction. Natural polyphosphate is composed of various salts, such as Ca, Mg, Na, and K salts, and the action of each salt and their differences are not known.

By using artificially produced polyphosphate, the inventors have found that the Ca salt has a platelet aggregating action, whereas the other metal salts such as Na salt do not produce platelet aggregation. Furthermore, the inventors have found that, by oral administration, an amorphous substance composed of a Ca salt of polyphosphate causes platelet aggregation in damaged gastrointestinal mucosa, thereby promoting healing of the damaged gastrointestinal mucosa.

The platelet aggregating action of polyphosphate can be evaluated as described in the Examples below. For example, the platelet aggregating action can be evaluated in vitro by adding a polyphosphate salt (polyphosphate nanoparticles, etc.) to platelet-rich plasma (PRP) and measuring the change in turbidness. The platelet aggregating action can also be evaluated in vivo by preparing an disease model animal, orally administering a polyphosphate salt, and observing the localization of CD61 (which is present on the platelet surface) at the affected site.

The platelet aggregating agent of the present invention may contain pharmacologically acceptable carriers and additives. Examples of such carriers and additives include excipients, binders, lubricants, solvents, disintegrants, solubilizing agents, suspending agents, emulsifiers, isotonic agents, stabilizers, preservatives, antioxidants, flavoring agents, coloring agents, buffers, and fluidity promoters, but are not limited thereto, and other conventionally used carriers and additives can be used as appropriate.

Specifically, examples of excipients include organic excipients such as sugars including lactose, glucose, and D-mannitol, starches, and celluloses including crystalline cellulose, and inorganic excipients such as calcium carbonate and kaolin.

Examples of binders include pregelatinized starch, gelatin, gum arabic, methyl cellulose, carboxymethyl cellulose, sodium carboxymethyl cellulose, crystalline cellulose, D-mannitol, trehalose, hydroxypropyl cellulose, hydroxypropyl methylcellulose, polyvinylpyrrolidone, and polyvinyl alcohol.

Examples of lubricants include stearic acid, fatty acid salts such as stearates, talc, and silicates.

Examples of solvents include purified water, physiological saline, and phosphate buffer solution.

Examples of disintegrants include low-substituted hydroxypropyl cellulose, chemically-modified cellulose and starches.

Examples of solubilizing agents include polyethylene glycol, propylene glycol, trehalose, benzyl benzoate, ethanol, sodium carbonate, sodium citrate, sodium salicylate, and sodium acetate.

Examples of suspending agents or emulsifiers include sodium lauryl sulfate, gum arabic, gelatin, lecithin, glycerol monostearate, polyvinyl alcohol, polyvinyl pyrrolidone, celluloses such as sodium carboxymethyl cellulose, polysorbates, and polyoxyethylene hydrogenated castor oil.

Examples of isotonic agents include sodium chloride, potassium chloride, sugars, glycerin, and urea.

Examples of stabilizers include polyethylene glycol, dextran sulfate sodium, and other amino acids.

Examples of preservatives include parahydroxybenzoates, chlorobutanol, benzyl alcohol, phenethyl alcohol, dehydroacetic acid, and sorbic acid.

Examples of antioxidants include the water-soluble antioxidants ascorbic acid, cysteine hydrochloride, sodium bisulfate, sodium metabisulfite, and sodium sulfite, the fat-soluble antioxidants ascorbyl palmitate, butylated hydroxyanisole (BHA), butylated hydroxytoluene (BHT), lecithin, propyl gallate, and α-tocopherol, and the metal chelating agents citric acid, ethylenediaminetetraacetic acid (EDTA), sorbitol, tartaric acid, and phosphoric acid.

Examples of flavoring agents include sweeteners and flavors used in common in the pharmaceutical field, and examples of coloring agents include colorants used in common in the pharmaceutical field.

The platelet aggregating agent of the present invention can be safely administered orally or parenterally (e.g., in the oral cavity, esophagus, stomach, small intestine, large intestine, and rectum) by making it a pharmaceutical preparation such as a tablet (including sugar-coated tablets, film-coated tablets, sublingual tablets, and orally disintegrating tablets), a powder, a granule, a capsule (including soft capsules and microcapsules), a liquid, a lozenge, a syrup, an emulsion, a suspension, an injection (e.g. subcutaneous injection, intramuscular injection, and intraperitoneal injection), an external preparation (e.g., transnasal administration formulation, transdermal formulation, and ointment), a suppository (e.g., rectal suppository and vaginal suppository), a foam, a pellet, a transnasal agent and a transpulmonary agent (inhalant). Preferably, the platelet aggregating agent of the present invention is administered orally, by suppository, or rectally, with oral administration being particularly preferable.

The platelet aggregating agent of the present invention may be a controlled-release formulation, such as a quick-release or sustained-release formulation. If it is an oral agent, it may be coated for masking, intestinal solubility, or durability, as necessary. Examples of coating materials used for coating include sugar-coating materials, water-soluble film-coating materials, enteric film-coating materials, and sustained-release film-coating materials.

The platelet aggregating agent of the present invention may be administered to humans or to non-human mammals. The dosage and method of administration are not particularly limited and can be determined as appropriate according to the condition, age, and the like of the individual to be administered.

The dosage of the platelet aggregating agent of the present invention is determined as appropriate by its intended use, route of administration, and the like. When administered to a human, for example, the dosage can be selected within a range of 0.05 mg/kg to 15 mg/kg of polyphosphate as a daily dose, preferably 0.05 mg/kg to 5 mg/kg, and more preferably 0.05 mg/kg to 2.5 mg/kg. Alternatively, it can be administered, for example, in the range of 3 to 900 mg/day, preferably 3 to 300 mg/day, and more preferably 3 to 150 mg/day, per patient, divided into one to several doses per day.

The polyphosphate of the present invention is composed of an amorphous substance composed of a polyphosphate Ca salt, which is the main body of the platelet aggregating action. Therefore, a higher platelet aggregating action can be expected compared to natural polyphosphate, which is a miscellaneous mixture of other metal salts and the like.

The platelet aggregating agent of the present invention may be used in combination with other agents as long as it does not undermine the purpose of the present invention. Examples of the medicaments that can be used in combination with the platelet aggregating agent of the present invention include agents commonly used in the treatment of inflammatory bowel diseases such as ulcerative colitis and Crohn's disease, such as 5-aminosalicylic acid (5 ASA) preparations including mesalazine and salazosulfapyridine; steroids preparations including prednisolone and methylprednisolone; anti-TNF-a preparations including infliximab and adalimumab; thiopurine preparations including mercaptopurine and azathioprine; and immunosuppressants including cyclosporine and tacrolimus. The time of administration of the platelet aggregating agent of the present invention and the concomitant drug is not limited, and they may be administered simultaneously or at different times.

### 4. Inflammatory Bowel Disease

As described above, by oral administration, the amorphous substance composed of a Ca salt of polyphosphate exerts a platelet aggregating action in damaged gastrointestinal mucosa, and promotes healing of the damaged gastrointestinal mucosa. Therefore, the amorphous polyphosphate of the present invention is useful for the prevention or treatment of inflammatory bowel disease (IBD).

"Inflammatory bowel disease" collectively refers to chronic or remitting/recurrent inflammatory diseases of the intestinal tract, and generally refers to two diseases: ulcerative colitis (UC) and Crohn's disease (CD). Ulcerative colitis is a diffuse non-specific inflammation of unknown cause, which produces erosion or ulceration of the mucosa of the large intestine. Crohn's disease is a general term for a chronic inflammatory disease of unknown cause, which causes chronic inflammation or ulceration of the mucosa of the large and small intestine.

The inventors have reported that polyphosphate has the effect of restoring the intestinal barrier function and preventing or improving inflammatory bowel disease. The amorphous polyphosphate of the present invention combines the above action of restoring the intestinal barrier function with the platelet aggregating effect unique to Ca salts, and therefore more effectively treat damaged gastrointestinal mucosa, which leads to the induction and maintenance of remission of inflammatory bowel disease. The present invention also provides a medicament for inducing/maintaining remission of inflammatory bowel disease, which contains such platelet aggregating agent.

### 5. Food/Drink

The amorphous polyphosphate of the present invention can be safely taken orally, and therefore can also be used as an ingredient in foods for specified health uses, foods for special dietary uses, dietary supplements, health foods, functional foods, foods for the sick, and the like.

### Examples

Hereinafter, the present invention is described specifically with Examples, but the present invention is not limited to these Examples.

### Example 1: Preparation of calcium polyphosphate particles

1.2 g of sodium long-chain polyphosphate (average chain length 450-700: Kamui Pharma) was added to 60 mL of purified water and dissolved. While measuring the pH of the dissolved aqueous solution of sodium long-chain polyphosphate using a pH meter (HORIBA F-55, pH electrode 9680S-10D) while stirring, a 1 N sodium hydroxide solution (manufactured by Wako Pure Chemical Industries) was added dropwise to adjust the pH of the aqueous solution to pH 10.

0.865 g of calcium chloride hydrate (Japanese Pharmacopoeia "For manufacture only", manufactured by Wako Pure Chemical Industries) was added to 7.5 mL of purified water and dissolved to prepare a calcium chloride aqueous solution. All of the prepared calcium chloride aqueous solution was added dropwise to the sodium long-chain polyphosphate aqueous solution at a drip rate of about 0.2 g/min using a peristaltic pump. The pH was consistently monitored during the dropping of the calcium chloride aqueous solution, and every time the pH shifted to the acidic side below pH 10, a 1 N sodium hydroxide solution was added dropwise to maintain the pH at pH 10. After all of the calcium chloride aqueous solution was added dropwise, stirring was continued for 4 hours at room temperature.

The obtained suspension was isolated preparatively to a centrifugal ultrafiltration filter unit (Amicon Ultra-15, nominal molecular weight cut off: 3 kDa, sample volume: 15 mL) and centrifuged at 4,000 rpm (3,040 × g) for 3 hours at 20°C using a centrifuge (KUBOTA Model 5911) to concentrate the solid content and desalt at the same time. To the filter device containing the concentrated precipitate, 2 mL of ethanol (99.5%, manufactured by Wako Pure Chemical Industries) was added to redisperse it, and then the mixture was centrifuged at 4,000 rpm (3,040 × g) for 1 hour at 20°C to remove the ethanol washing liquid. This washing operation with ethanol was repeated twice.

The filter device with the precipitate after washing with ethanol was placed in a vacuum dryer (DP-33, Yamato Scientific), and dried at 50°C for 12 hours after reducing the pressure by vacuum drawing. After drying, the solid content was passed through a sieve (aperture: 500 µm) to obtain 1.187 g of calcium polyphosphate particle powder. Assuming that 1 molar equivalent of sodium ion in the sodium polyphosphate is 100% replaced with a 1/2 molar equivalent of calcium ion, the yield of calcium polyphosphate particles was calculated to be 101.9%.

The average particle size and zeta potential of the prepared calcium polyphosphate particles were measured using a dynamic light scattering (DLS) particle size distribution analyzer (Zetasizer model Nano-ZS, Marvern Instruments). The average particle size was 1252 nm and the zeta potential was -36.2 mV.

### Example 2: Acute enterocolitis mouse model

### 1. Materials and methods

### 1. 1 Preparation of acute enterocolitis mouse model and therapeutic trial

Six- to 8-week-old male BALB/c mice (Charles River Laboratories Japan) were treated with 2% dextran sulfate sodium (MP Biomedicals) via free access to water for 5 days. Sodium polyphosphate and calcium polyphosphate were dissolved in phosphate buffer (pH 7.4) and orally administered to the mice once a day from the first day, and on day 7, the mice were euthanized and sampled.

### 1. 2 Evaluation of Intestinal Tract Length

The abdomen of the mice was picked up with tweezers and opened with dissecting scissors, the femoral and pelvic joints were removed, and the lower large intestine leading to the anal region was cut with scissors. The large intestine was then carefully detached by picking up with tweezers the tip of the large intestine cut with scissors, and the top of the cecum was cut with scissors. The excised large intestine was placed on a Kim towel and the large intestine length was measured with a ruler.

### 1.3 RT-PCR

The excised intestinal tract was opened with scissors, and the mucosa of the large intestine was detached with a glass slide, then added to 500 µL of TRIzol. After centrifuging at 15,000 rpm for 5 minutes at 4°C, the supernatant was transferred to a 1.5 mL Eppendorf tube. 0.1 mL of chloroform was added to the tube, then the mixture was mixed with a vortex mixer and centrifuged 15,000 rpm for 5 minutes at 4°C. After the centrifugation, 250 µL of the aqueous layer was transferred to a new 1.5 mL Eppendorf tube. 250 µL of isopropanol was added to wash the pellet, then 0.5 mL of 70% ethanol was added to wash the pellet. The pellet was dissolved in 200 µL of sterile water, and the RNA was purified using a RNeasy Mini Kit (Qiagen). The reverse transcription reaction was performed using the high-capacity cDNA reverse transcription kit (Applied Biosystems) in accordance with the product documentation. Using the recovered cDNA as a template, Taqman RT-PCR was performed using primers specific to various inflammation-related cytokines, and spectral data were obtained by the Applied Biosystems 7300 Real Time PCR system (Applied Biosystems). The spectrum of 18S rRNA was obtained using the same cDNA as a template and standardized. The spectral data were used to quantify the expression levels relative to the PBS-treated group set as 1 by the ΔΔCt method.

### 1.4 Evaluation of Histological Severity

The excised intestinal tract was opened with scissors to prepare an intestinal roll, and the tissue was fixed with a 10% formalin solution. After embedding it in paraffin, the fixed tissue was thinly cut into 4 µm slices and stained with hematoxylin-eosin. Histological severity was evaluated based on the evaluation items of Berg et al. (Berg DJ. et al. J Clin Invest, 1998).

**[Table 1]**

| Evaluation of Histological Severity | |
|---|---|
| Grade 0: | Normal Tissue |
| Grade 1: | One or a few multifocal mononuclear cell infiltrates in the lamina propria, accompanied by no depletion of mucus from goblet cells. Grade 2: Mild inflammatory cell infiltration in the lamina propria composed primarily of mononuclear cells with a few neutrophils. |
| Grade 3: | Inflammatory cell infiltration often involved the submucosa, and mucin depletion was seen |
| Grade 4: | Inflammatory cell infiltration involved the muscularis, and mucin production almost disappeared. Crypt abscesses and ulcers were present. |

### 1.5 CD61 Immunohistochemical Staining

Paraffin-embedded intestinal tract sections were thinly cut into 4 µm slices to prepare tissue slides. Deparaffinization was performed, then antigen activation by boiling in citric acid buffer (pH 6.0) at 121°C for 20 minutes. This was reacted in an anti-CD61 antibody (Cell Signaling Technologies) solution at 4°C overnight while blocking nonspecific reactions with SuperBlock T20 (PBS) (Thermo Fisher Scientific). The tissue was washed 3 times for 5 minutes in PBS, treated with ImmPRESS Universal Reagent Anti-Rabbit IgG (VECTOR Laboratories), and then the tissue was washed 3 times for 5 minutes in PBS. Color was produced using ImmPACT DAB Peroxidase Substrate Kit (VECTOR Laboratories), then nuclear staining was performed in hematoxylin solution, followed by encapsulation. CD61-positive areas were extracted using Image J, a free image analysis software, and quantified by measuring the area of the positive areas.

### 2. Results for Acute Enterocolitis Mouse Model

### 2.1 Evaluation of Intestinal Tract Length and Histological Severity (Figures 1 to 3)

Figure 1 shows the intestinal tract lengths of each group: PBS-treated group, calcium polyphosphate (PPA-Ca (1 µg/mouse)) treated group, PPA-Ca (5 µg/mouse) treated group, and tacrolimus (TAC (60 µg/mouse)) treated group. The histological severity scores for each group are also shown in Figure 2. The PPA-Ca-treated groups (1 µg and 5 µg/mouse) had significantly longer intestinal tracts than the PBS-treated group. Similarly, the histological severity was significantly lower in the PPA-Ca-treated groups than in the PBS-treated group, indicating a therapeutic effect of calcium polyphosphate in the acute enterocolitis mouse model.

The effect of sodium polyphosphate (PPA-Na (5 µg/mouse)) was compared to that of calcium polyphosphate (PPA-Ca (5 µg/mouse)). The PPA-Ca-treated group had significantly longer intestinal tracts than the PBS-treated group, but no significant difference from the PBS-treated group was found for the PPA-Na-treated group (Figure 3), indicating that calcium polyphosphate has a stronger therapeutic effect than sodium polyphosphate in the acute enterocolitis mouse model.

### 2. 2 Expression of Inflammatory Cytokines (Figures 4 and 5)

Figure 4 shows the results of RT-PCR analysis of the expression of inflammatory cytokines (TNFα, IL-1β, IFNγ, and IL6) in each group: PBS-treated group, calcium polyphosphate (PPA-Ca (1 µg/mouse)) treated group, PPA-Ca (5 µg/mouse) treated group, and tacrolimus (TAC (60 µg/mouse)) treated group. In the PPA-Ca-treated groups (1 µg and 5 µg/mouse), the expressions of TNFα, IL-1β, IFNγ, and IL6 were all significantly lower compared to the PBS-treated group. The inhibitory effect of calcium polyphosphate on inflammatory cytokine expression was shown in the acute enterocolitis mouse model.

The effect of sodium polyphosphate (PPA-Na (5 µg/mouse)) was compared to that of calcium polyphosphate (PPA-Ca (5 µg/mouse)) (Figure 5). In the PPA-Ca-treated group, the expressions of IL-1β and TNFα, were significantly lower than the PBS-treated group, and the expression levels of IFNy and IL6 were also reduced to the same level as the TAC-treated group (60 µg/mouse). On the other hand, no decrease in the expression of any of the inflammatory cytokines was observed in the PPA-Na-treated group at that dose, indicating that calcium polyphosphate suppressed the expression of inflammatory cytokines more strongly than sodium polyphosphate in the acute enterocolitis mouse model.

### 2. 3 CD61-Positive Area (Figure 6)

Figure 6 shows a graph comparing CD61-positive areas in the intestinal tissue of an acute enterocolitis mouse model. Compared to the no-treatment group (Control), the CD61-positive area was significantly larger in the DSS-administered group (DSS, PBS), but compared to the DSS-administered group (DSS, PBS), the CD61-positive area was significantly larger in the PPA-Ca-treated group (DSS, PPA-Ca: 5 µg/mouse). Since CD61 is expressed on the platelet surface, the CD61-positive area expands when platelet aggregation occurs. The above results indicate that oral administration of PPA-Ca highly promotes platelet aggregation on the inflamed intestinal mucosa.

### Example 3: Platelet Aggregation Assay

### 1. Materials and methods

After obtaining consent for the study, blood was collected using a blood collection tube with 3.2% sodium citrate buffer, and platelet-rich plasma (PRP) was recovered by centrifuging at 800 g for 10 minutes. The obtained PRP was then reacted on a 96-well plates with sodium polyphosphate and calcium polyphosphate each at a concentration of 100 µg/100 µL PRP, and based on the platelet aggregation assay by Chan MV et al. (Chan MV et al. Platelets, 2018), the absorbance (405 nm) was measured every 10 seconds using an EnSpire Multimode Plate Reader (Perkinelmer) and the changes over time of PRP turbidity was analyzed. For analysis, the absorbance at the start was set to 1 and the relative values were calculated to analyze the changes over time.

### 2. Results (Figure 7)

Almost no difference could be observed between the change in absorbance when sodium polyphosphate (PPA-Na) was added and that of the negative control, no-addition. On the other hand, the change in absorbance when calcium polyphosphate (PPA-Ca) was added indicated a turbidity comparable to the positive control, adenosine diphosphate (ADP). This indicates that PPA-Ca exerts a pronounced platelet aggregating effect, while no platelet aggregating effect was found for PPA-Na.

### Example 4: Wound Healing Assay

### 1. Materials and methods

Platelet-rich plasma was recovered by the same method as above, to which sodium polyphosphate and calcium polyphosphate were added at 1 mg/ml each and reacted for 20 minutes at room temperature. Platelets were then removed from PRP by centrifuging at 2,000 g for 10 minutes. The recovered plasma was added to a Vivaspin 3K column (GE Healthcare) and centrifuged at 3,040 × g to recover relatively low molecular weight plasma components from which platelets and polyphosphate particles were removed. A linear wound was drawn using the tip of a P200 tip on a human colonic epithelial cell line (HCEC-1CT cells) cultured to confluence in a 12-well plate, to which the relatively low molecular weight plasma components recovered above were added. The degree of wound healing was assessed by taking photographs over time and measuring the width of the linear wound.

### 2. Results (Figure 8)

Figure 8 shows the time course of wound width. For the plasma components recovered from PRP to which sodium polyphosphate (PPA-Na) was added, no significant difference in the degree of wound healing was observed compared to the negative control, no addition to PRP. On the other hand, with the plasma components recovered from PRP to which calcium polyphosphate (PPA-Ca) was added, the degree of wound healing was significantly improved.

As the platelets and polyphosphate particles were removed from the plasma components added to the cells in the assay, this indicates that liquid factors resulting from platelet aggregation by calcium polyphosphate promoted wound healing.

### 3. Discussion

It was shown that calcium polyphosphate aggregates platelets in the intestinal mucosa of an enterocolitis mouse model (acute), and furthermore, that liquid factors resulting from platelet aggregation promote intestinal epithelial cell repair. On the other hand, no platelet aggregating effect or effect of promoting intestinal epithelial cell repair based thereon was observed for sodium polyphosphate.

### Example 5: Chronic enterocolitis mouse model

### 1. Materials and methods

### 1. 1 Preparation of chronic enterocolitis mouse model and therapeutic trial

Six- to 8-week-old male C57BL/6J mice (Charles River Laboratories Japan) had free access to water containing 2% dextran sulfate sodium (MP Biomedicals) for 5 days. The mice were then allowed free access to distilled water until day 35. Sodium polyphosphate (1 µg/mouse) and calcium polyphosphate (1 µg/mouse) were dissolved in phosphate buffer (pH 7.4) and orally administered to the mice once a day from day 25, and on day 35, the mice were euthanized and sampled.

### 1. 2 Evaluation of Intestinal Tract Length

The evaluation was performed in accordance with the method described in Example 2.

### 1. 3 RT-PCR

The RT-PCR was performed in accordance with the method described in Example 2.

### 1. 4 Evaluation of Histological Severity

The evaluation was performed in accordance with the method described in Example 2.

### 2. Results

### 2. 1 Evaluation of Intestinal Tract Length and Histological Severity (Figures 9 and 10)

Figure 9 shows the lengths of the intestinal tract in each group: PBS-treated group, calcium polyphosphate (PPA-Ca (5 µg/mouse)) treated group, and PPA-Na (5 µg/mouse) treated group. In the chronic enterocolitis model, the PPA-Ca-treated group had significantly longer intestinal tracts than the PBS-treated group (p<0.05), and a certain improvement (p=0.07) was also shown in the PPA-Na-treated group. The histological severity scores for each group are also shown in Figure 10. A significant improvement in histological severity was observed in both the PPA-Ca-treated group and PPA-Na-treated group (p<0.05).

### 2. 2 Expression of Inflammatory Cytokines (Figure 11)

Figure 11 shows the results of RT-PCR analysis of the expression of inflammatory cytokines (TNFα, IL-1β,and IFNy). In the PPA-Ca-treated group, the expression levels for any of the inflammatory cytokines were significantly lower (p<0.05) compared to the PBS-treated group. In contrast, although no significant difference with the PBS-treated group was observed for the PPA-Na-treated group, a certain decrease in the expression of IFNy was observed (p=0.06).

### 3. Discussion

Calcium polyphosphate was found to be useful for both chronic and acute enterocolitis. When compared to sodium polyphosphate, even a smaller dosage was sufficient to exert an effect of promoting healing of the damaged mucosa, and the superiority of calcium polyphosphate was particularly pronounced in acute enterocolitis. These differences were considered to be due in part to the platelet aggregating action in the damaged mucosa, which is not observed with sodium polyphosphate, and the resulting promotion of mucosal healing by the liquid factors.

### Industrial Applicability

The present invention can act on damaged gastrointestinal mucosa and promote a platelet aggregation, thereby promoting mucosal healing in inflammatory bowel disease. Therefore, it is useful for the prevention or treatment of inflammatory bowel disease.

All publications, patents and patent applications cited in the present specification are hereby incorporated by reference in their entirety.

## Claims

1. A platelet aggregating agent comprising amorphous polyphosphate as an active ingredient, wherein the polyphosphate is a Ca salt of polyphosphate.

2. The platelet aggregating agent according to claim 1, which promotes platelet aggregation in damaged gastrointestinal mucosa.

3. The platelet aggregating agent according to claim 1 or 2, which is administered to a patient with inflammatory bowel disease.

4. The platelet aggregating agent according to any one of claims 1 to 3, which is orally administered.

5. The platelet aggregating agent according to any one of claims 1 to 4, which promotes healing of damaged gastrointestinal mucosa.

6. The platelet aggregating agent according to any one of claims 1 to 5, wherein the amorphous polyphosphate is in nanoparticle form.

7. The platelet aggregating agent according to claim 6, wherein a zeta potential of the polyphosphate nanoparticles is 0 mV or less.

8. The platelet aggregating agent according to claim 6 or 7, wherein an average particle size of the polyphosphate nanoparticles is 10 nm to 3000 nm.
